(19) Europäisches Patentamt
European Patent Office
Office européen des brevets

(11) Veröffentlichungsnummer : **0 133 530**
**B1**

(12) # EUROPÄISCHE PATENTSCHRIFT

(45) Veröffentlichungstag der Patentschrift : **15.03.89**

(21) Anmeldenummer : **84109019.4**

(22) Anmeldetag : **30.07.84**

(51) Int. Cl.$^4$ : **C 07 D471/04**, A 61 K 31/435 // (C07D471/04, 221:00, 221:00)

(54) **1,6-Naphthyridinon-Derivate, Verfahren zu deren Herstellung und diese enthaltende Arzneimittel.**

(30) Priorität : **30.07.83 DE 3327650**

(43) Veröffentlichungstag der Anmeldung : **27.02.85 Patentblatt 85/09**

(45) Bekanntmachung des Hinweises auf die Patenterteilung : **15.03.89 Patentblatt 89/11**

(84) Benannte Vertragsstaaten : **AT BE CH DE FR GB IT LI LU NL SE**

(56) Entgegenhaltungen :
**FR--A-- 2 485 535**
**US--A-- 4 304 914**
**CHEMICAL ABSTRACTS, Band 103, Nr. 21, 1985, Seite 682, Spalte 2, Zusammenfassungsnr. 178253t, Columbus, Ohio, US; M. BALOGH et al.: "Bactericidal and fungicidal 1,6-naphthyridine derivatives and salts"; & HU - A - 33 804 (CHINOIN GYOGYSZER ES VEGYESZETI TERMEKEK GYARA RT.) 28.12.1984**

(73) Patentinhaber : **GÖDECKE AKTIENGESELLSCHAFT**
**Salzufer 16**
**D-1000 Berlin 10 (DE)**

(72) Erfinder : **Satzinger, Gerhard, Dr.**
**Im Mattenbühl 7**
**D-7809 Denzlingen (DE)**
Erfinder : **Hartenstein, Johannes, Dr.**
**Fohrenbühl 23**
**D-7801 Stegen-Wittental (DE)**
Erfinder : **Mannhardt, Karl, Dr.**
**Pfauenstrasse 14**
**D-7807 Elzach-Oberprechtal (DE)**
Erfinder : **Kleinschroth, Jürgen, Dr.**
**Freiburger Strasse 13**
**D-7809 Denzlingen (DE)**
Erfinder : **Herrmann, Manfred, Dr.**
**Wolfweg 25**
**D-7811 St. Peter (DE)**
Erfinder : **Fritschi, Edgar, Dr.**
**Am Scheuerwald 2**
**D-7811 St. Peter (DE)**
Erfinder : **Tauschel, Horst-Dietmar, Dr.**
**Hinterdorfstrasse 29**
**D-7637 Ettenheim (DE)**
Erfinder : **Wagner, Bernd, Dr.**
**Am Lossele 5**
**D-7809 Denzlingen (DE)**
Erfinder : **Wolf, Günter, Dr.**
**Wildtalstrasse 40**
**D-7800 Freiburg (DE)**

**EP 0 133 530 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft neue 1,6-Naphthyridinon-derivate der allgemeinen Formel I

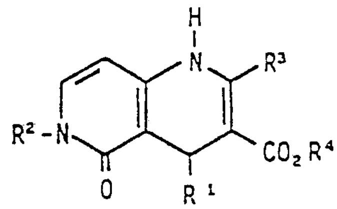

(I)

in welcher $R^1$ einen Phenylrest, der in 2- und/oder 2-Stellung durch Halogenatome, wie Fluor, Chlor oder Brom, durch Nitro-, Trifluormethyl-, Methoxy-, Difluormethoxy- oder Cyanogruppen oder Niederalkylaminogruppen, bevorzugt Dimethyl- oder Diethylaminogruppen substituiert sein kann, oder einen unsubstituierten Pyridyl- oder Thienylrest, $R^2$ Wasserstoff, eine Alkyl- oder Alkoxyalkylgruppe mit bis zu 6 Kohlenstoffatomen oder eine Alkylaminoalkylgruppe der allgemeinen Formel II

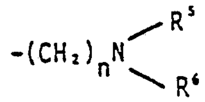

(II)

in welcher $R^5$ und $R^6$ gleich oder verschieden sein können und eine geradkettige oder verzweigte Alkylgruppe mit 1-4 C-Atomen darstellen oder gemeinsam eine Alkylengruppe mit 4-6 C-Atomen bilden, n die Zahl 2 oder 3, $R^3$ eine Aminogruppe oder eine Methyl- oder Ethylgruppe und $R^4$ Wasserstoff oder einen geradkettigen, verzweigten oder cyclischen Alkyl- oder Alkoxyalkylrest mit bis zu 6 Kohlenstoffatomen bedeutet ;
sowie gegebenenfalls deren pharmakologisch unbedenklichen Salze.

Die Erfindung betrifft weiterhin ein Verfahren zur Herstellung von 1,6-Naphthyridinon-Derivaten der allgemeinen Formel I, das dadurch gekennzeichnet ist, daß man entweder

a) 2,4-Dihydroxypyridin mit einer Verbindung der allgemeinen Formel III

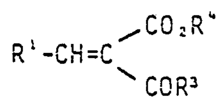

(III)

in welcher $R^1$, $R^3$ und $R^4$ die obengenannte Bedeutung haben,
in Gegenwart von Ammoniak umsetzt und die so erhaltenen Verbindungen der allgemeinen Formel IV

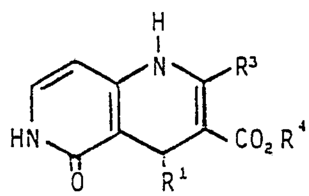

(IV)

in welcher $R^1$, $R^3$ und $R^4$ die obengenannte Bedeutung haben,
gewünschtenfalls in an sich bekannter Weise mit einer Verbindung der allgemeinen Formel V

$$X—R^{2'} \qquad (V)$$

in welcher $R^{2'}$ eine Alkylgruppe oder eine Alkylaminogruppe der allgemeinen Formel II darstellt und X ein Halogenatom bedeutet, umsetzt oder

b) ein 1,4-Dihydropyridin der allgemeinen Formel VI

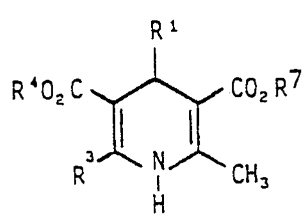

(VI)

in welcher $R^1$, $R^3$ und $R^4$ die obengenannte Bedeutung haben und $R^7$ einen Alkylrest mit 1-4 Kohlenstoffatomen bedeutet
in Gegenwart einer Base mit s-Triazin umsetzt und die erhaltene Verbindung der allgemeinen Formel IV anschließend gewünschtenfalls mit einer Verbindung der allgemeinen Formel V alkyliert oder aminoalkyliert oder

c) ein 1,4-Dihydropyridin der allgemeinen Formel VI mit einem Dialkylformamid-dialkylacetal der allgemeinen Formel VII

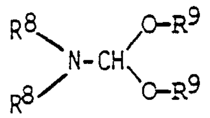

(VII)

in welcher $R^8$ gleich oder verschieden sein kann und eine Methyl- oder Ethylgruppe bedeutet und die Reste $R^9$ jeweils eine Alkylgruppe mit bis zu 4 Kohlenstoffatomen oder gemeinsam eine Alkylengruppe mit bis zu 3 Kohlenstoffatomen bedeuten
und die erhaltene Verbindung der allgemeinen Formel VIII

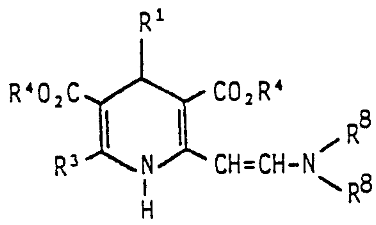

(VIII)

in welcher $R^1$, $R^3$, $R^4$ und $R^8$ die obengenannte Bedeutung haben, mittels Ammoniak in eine Verbindung der allgemeinen Formel IV überführt und diese gewünschtenfalls mit einer Verbindung der allgemeinen Formel V alkyliert oder aminoalkyliert und die erhaltenen Verbindungen der allgemeinen Formel I ggf. durch Umsetzung mit organischen oder anorganischen Säuren in deren pharmakologisch verträglichen Salze überführt.

Aus der US-A-4 304 914 sind zwar bereits Naphthyridinonderivate mit pharmakologischer Wirksamkeit bekannt geworden. Diese sind jedoch in 6,7-Stellung arylsubstituiert und besitzen eine cardiotonische und antihypertensive Wirksamkeit, während die erfindungsgemäßen Derivate eine gefäßspasmolytische und eine thrombocytenaggregationshemmende Wirkung aufweisen. Bevorzugt sind für $R^5$ und $R^6$ Methyl- und Ethylgruppen oder gemeinsam der Pentamethylenrest.

Bevorzugt sind weiter Verbindungen der allgemeinen Formel I, in welcher $R^2$ ein Wasserstoffatom oder eine Alkylgruppe mit bis zu 4 Kohlenstoffatomen bedeutet.

Die Verbindungen der allgemeinen Formel I weisen wertvolle pharmakologische Eigenschaften bei günstiger allgemeiner Verträglichkeit auf.

Aufgrund ihrer gefäßspasmolytischen und thrombozytenaggregationshemmenden Wirkungen sind sie vor allem bei cerebralen, cardialen und peripheren Gefäßerkrankungen wie myokardialer Ischämie, bei cerebralem Infarkt, pulmonalen Thrombosen und bei Arteriosklerose und anderen stenotischen Erscheinungen indiziert, insbesondere weil im Vergleich mit bekannten Präparaten ähnlicher Wirkungsweise negativ inotrope Nebeneffekte weitgehend fehlen. Die 1,6-Naphthyridinon-Derivate der vorliegenden Erfindung sind daher wertvolle Arzneimittel für die Bekämpfung der Herz-Kreislauf-Mortalität, die in Deutschland zur Zeit bei über 50 % aller Todesfälle liegt. Ein weiterer Gegenstand der vorliegenden Erfindung sind daher 1,6-Naphthyridinon-Derivate der allgemeinen Formel I als Wirkstoff enthaltende Arzneimittel.

Die Verbindungen der allgemeinen Formel III sind bekannt oder können nach literaturbekannten Verfahren hergestellt werden [Org. Reactions, Vol. 15 (1967) S. 204 ff]. 2,4-Dihydroxypyridin ist Handelsprodukt.

Die Reaktion a) wird vorzugsweise in inerten organischen Lösungsmitteln, insbesondere niederen Alkoholen wie z. B. Methanol, Ethanol oder Isopropanol durchgeführt. Es ist auch zweckmäßig, bei höheren Temperaturen, vorzugsweise bei Siedetemperatur des verwendeten Lösungsmittels zu arbeiten. Die Reaktionsprodukte lassen sich mit bekannten Trennverfahren, wie Kristallisation und/oder Chromatographie isolieren und reinigen.

Das Verfahren a) ist chemisch eigenartig, da es als überraschend anzusehen ist, daß 2,4-Dihydroxypyridin in der beschriebenen Weise reagiert.

Die für das Verfahren b) eingesetzten 1,4-Dihydropyridine der allgemeinen Formel IV sind bekannt [vgl. z. B. Chem. Rev. 82 (1982) S. 223] oder sie können in analoger Weise hergestellt werden.

Die Reaktion von s-Triazin mit den Verbindungen der allgemeinen Formel VI muß ebenfalls als überraschend angesehen werden, da aus der Literatur [Chem. Rev. 82 (1982)] hervorgeht, daß bei der

Behandlung von 1,4-Dihydropyridinen mit starken Basen wie Natriumhydrid das Proton am Stickstoffatom unter Bildung eines Natriumamids entfernt wird, welches dann z. B. mit Alkylhalogeniden zu N-Alkylderivaten weiterreagiert. Im vorliegenden Fall findet die erwartete Reaktion nicht statt, sondern es erfolgt an der Methylgruppe eine Aminomethenylierung, die zu den Verbindungen der Formel IV führt.

Zur Durchführung der Reaktion wird das 1,4-Dihydropyridinderivat mit s-Triazin in einem inerten organischen Lösungsmittel in Gegenwart starker Basen wie z. B. Alkalialkoholaten oder Natriumhydrid in einem inerten organischen Lösungsmittel auf Temperaturen von 50-160 °C, vorzugsweise 100-150 °C erhitzt. Als Lösungsmittel eignen sich hier vor allem polare Lösungsmittel wie Dimethylsulfoxid, Dimethylformamid, Ethylenglykoldimethylether oder niedere Alkohole wie Ethanol.

Zur Durchführung der Reaktion nach Verfahrensvariante c) wird das entsprechende 1,4-Dihydropyridinderivat mit einer äquivalenten oder überschüssigen Menge Dialkylformamiddialkylacetal, vorzugsweise in Gegenwart eines aprotischen Lösungsmittels wie Dimethylformamid, Dimethylsulfoxid oder Hexamethylphosphorsäuretriamid, unter Erwärmen umgesetzt. Als geeignete Formamidacetale kommen vor allem Dimethylformamiddimethylacetal und Dimethylformamiddiethylacetal in Frage.

Das nach der Verfahrensvariante c) erhaltene Zwischenprodukt der allgemeinen Formel VII wird durch Reaktion mit Ammoniak in Gegenwart eines, vorzugsweise protischen Lösungsmittels bei Raumtemperatur oder bei höherer Temperatur, bevorzugt bei der Siedetemperatur des verwendeten Lösungsmittels in Verbindungen der allgemeinen Formel IV übergeführt. Als Lösungsmittel sind vor allem niedere Alkohole, wie Methanol oder Ethanol geeignet. Die weitere Umsetzung mit Verbindungen der allgemeinen Formel V wird nach allgemein bekannten Methoden vorzugsweise in Gegenwart eines Halogenwasserstoffakzeptors durchgeführt. Es hat sich gezeigt, daß die Reaktion mit hoher Regioselektivität abläuft und, daß bei der Umsetzung überraschenderweise die an sich zu erwartende O-Alkylierung analog den Verhältnissen bei der Alkylierung von Pyridonen praktisch vollständig unterdrückt wird.

Saure oder basische Verbindungen der allgemeinen Formel I, welche für $R^4$ ein Wasserstoffatom bzw. für $R^2$ eine Alkylaminogruppe II aufweisen, überführt man zum Zwecke der Reinigung und aus galenischen Gründen bevorzugt in kristalline, pharmakologisch verträgliche Salze.

Für den Fall, daß $R^4$ ein Wasserstoffatom darstellt, lassen sich mit Basen wie z. B. Natriumhydroxid oder Natriumcarbonat entsprechende Salze der Alkali- oder Erdalkalimetalle herstellen. Wenn der Rest $R^2$ oder $R^3$ basischen Charakter aufweist, werden Salze in üblicher Weise durch Neutralisation der Basen mit entsprechenden anorganischen oder organischen Säuren erhalten. Als Säuren kommen z. B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure oder Bernsteinsäure in Frage.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält.

Derartige Zusätze sind z. B. Tartrat- und Citrat-Puffer, Ethanol, Komplexbildner (wie Äthylendiamintetraessigsäure und deren nichttoxische Salze) sowie hochmolekulare Polymere (wie flüssiges Polyäthylenoxid) zur Viskositätsregulierung. Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat, Magnesiumstearat, tierische und pflanzliche Fette, feste hochmolekulare Polymere (wie Polyäthylenglykol) ; für orale Applikation geeignete Zubereitungen können gewünschtenfalls zusätzliche Geschmacks- und/oder Süßstoffe enthalten.

Die enteral verabreichten Einzeldosen liegen im Bereich von etwa 5 bis 250 mg, vorzugsweise 20-100 mg. Parenteral werden etwa 1 bis 20 mg gegeben.

Die folgenden Beispiele sollen die Erfindung näher erläutern :

## Beispiel 1

1,4,5,6-Tetrahydro-2-methyl-4-(3-nitrophenyl)-5-oxo-1,6-naphthyridin-3-carbonsäureethylester

Variante a)

Eine Suspension von 2,4 g (9 mMol) 3-Nitrobenzylidenacetessigsäureethylester und 1,0 g (9 mMol) 2,4-Dihydroxypyridin in 25 ml Ethanol wird mit Ammoniakgas bei Raumtemperatur gesättigt. Unter weiterem Durchleiten von gasförmigem Ammoniak wird 5 Stunden zum Sieden erhitzt. Nach Abziehen des Lösungsmittels im Vakuum wird der Rückstand in 50 ml Chloroform unter Erwärmen gelöst. Die beim Abkühlen ausfallenden Kristalle werden abgesaugt und durch Kristallisation aus Ethenol/Ethylacetat 1 : 1 gereinigt. Man erhält 1,4,5,6-Tetrahydro-2-methyl-4-(3-nitrophenyl)-5-oxo-1,6-naphthyridin-3-carbonsäureethylester als gelbe Nadeln vom Schmp. 285 °C (Z).

Variante b)

Zu einer Suspension von 4,95 g (165 mMol) Natriumhydrid (80 %ig in Paraffinöl) in 75 ml trockenem Dimethylformamid wird unter Stickstoffatmosphäre eine Lösung von 56,5 g (150 mMol) 1,4-Dihydro-2,6-dimethyl-4-(3-nitrophenyl) pyridin-3,5-dicarbonsäurediethylester in 250 ml Dimethylformamid getropft. Bei Nachlassen der Gasentwicklung wird kurzzeitig (ca. 10 min) auf 60 °C erwärmt und anschließend werden 12,2 g (150 mMol) s-Triazin in 250 ml Dimethylformamid zugetropft. Die Reaktionsmischung wird 16 Stunden zum Sieden erhitzt, nach dem Abkühlen filtriert und im Vakuum eingeengt. Der dunkle Rückstand wird in 400 ml n-Hexan aufgekocht. Das ungelöste Rohprodukt wird nach Abdekantieren des n-Hexans in 500 ml heißem Ethanol suspendiert und auf eine Säule mit Kieselgel aufgetragen. Anschließend wird mit Methylenchlorid/methanol · Ammoniak (9 : 1 V/V) eluiert und die gewünschte Fraktion zur weiteren Reinigung zweimal aus Ethanol/Ethylacetat (1 : 1 V/V) umkristallisiert.

Man erhält 1,4,5,6-Tetrahydro-2-methyl-4-(3-nitrophenyl)-5-oxo-1,6-naphthyridin-3-carbonsäureethylester in Form gelber Nadeln vom Schmp. 285 °C (Z).

Beispiel 2

1,4,5,6-Tetrahydro-6-(3-dimethylaminopropyl)-2-methyl-4-(3-nitrophenyl)-5-oxo-1,6-naphthyridin-3-carbonsäureethylester

6,9 g (19,4 mMol) 1,4,5,6-Tetrahydro-2-methyl-4-(3-nitrophenyl)-5-oxo-1,6-naphthyridin-3-carbonsäureethylester, hergestellt gemäß Beispiel 1, und 2,4 g (19,4 mMol) N,N-Dimethylaminopropylchlorid werden mit 10,7 g (77,6 mMol) Kaliumcarbonat in 500 ml Aceton 48 Stunden zum Sieden erhitzt. Es wird im Vakuum zur Trockne eingeengt, der Rückstand in 500 ml Wasser und 500 ml Methylenchlorid aufgenommen und die organische Phase mit 500 ml Wasser gewaschen und über Natriumsulfat getrocknet. Das nach Abziehen des Lösungsmittels i. V. erhaltene braune Rohprodukt wird zweimal aus Acetonitril/Ethanol umkristallisiert. Man erhält 1,4,5,6-Tetrahydro-6-(3-dimethylaminopropyl)-2-methyl-4-(3-nitrophenyl)-5-oxo-1,6-naphthyridin-3-carbonsäureethylester in Form gelber Nadeln vom Schmp. 212-213 °C.

Beispiel 3

1,4,5,6-Tetrahydro-4-(3-chlorphenyl)-2-methyl-5-oxo-1,6-naphthyridin-3-carbonsäureethylester

6,0 g (14,3 mMol) 1,4-Dihydro-4-(3-chlorphenyl)-2-(2-dimethylaminoethenyl)-6-methylpyridin-3,5-dicarbonsäurediethylester werden in einer Mischung aus 60 ml konzentrierter wäßriger Ammoniaklösung und 60 ml Ethanol 6 Stunden zum Sieden erhitzt. Nach dem Abkühlen wird die Lösung im Vakuum auf die Hälfte des Volumens eingeengt. Es wird dann Wasser zugesetzt und zweimal mit Chloroform extrahiert. Die Chloroformlösung wird mit wenig Wasser gewaschen und über Natriumsulfat getrocknet. Der nach Abdestillieren des Lösungsmittels erhaltene Rückstand wird an Kieselgel mit Methylenchlorid/methanol · Ammoniak (9 : 1, V/V) chromatographiert und aus Methanol umkristallisiert. Man erhält 1,4,5,6-Tetrahydro-4-(3-chlorphenyl)-2-methyl-5-oxo-1,6-naphthyridin-3-carbonsäureethylester in Form beiger Kristalle vom Schmp. 152-155 °C (Z).

Der als Ausgangsprodukt verwendete 1,4-Dihydro-4-(3-chlorphenyl)-2-(2-dimethylaminoethenyl)-6-methylpyridin-3,5-dicarbonsäurediethylester wird wie folgt hergestellt:

Eine Lösung von 36,4 g (0,1 Mol) 1,4-Dihydro-4-(3-chlorphenyl)-2,6-dimethyl-pyridin-3,5-dicarbonsäurediethylester und 14,7 g (0,1 Mol) Dimethylformamiddiethylacetal in 100 ml trockenem Dimethylformamid wird unter Stickstoffatmosphäre 16 Stunden zum Sieden erhitzt. Nach dem Abkühlen wird das Lösungsmittel im Vakuum abdestilliert und der Rückstand in Toluol und Wasser aufgenommen und ausgeschüttelt. Die Toluollösung wird ein zweites Mal mit Wasser gewaschen und über Natriumsulfat getrocknet. Nach dem Filtrieren wird die Lösung i. V. eingedampft, und der Rückstand durch Verreiben mit Petrolether zur Kristallisation gebracht. Durch zweimaliges Umkristallisieren aus Diisopropylether erhält man gelbe Kristalle vom Schmp. 131-132 °C.

Beispiel 4

1,4,5,6-Tetrahydro-2,6-dimethyl-4-(2-methoxyphenyl)-5-oxo-1,6-naphthyridin-3-carbonsäureethylester

Zu einer Lösung von 0,4 g (17,4 mMol) Natrium in 80 ml absolutem Ethanol gibt man unter Rühren bei Raumtemperatur 5,7 g (16,8 mMol) 1,4,5,6-Tetrahydro-4-(2-methoxyphenyl)-2-methyl-5-oxo-1,6-naphthyridin-3-carbonsäureethylester. Sobald sich eine klare Lösung gebildet hat, werden bei Raumtemperatur 2,4 ml (25,3 mMol) Dimethylsulfat zugetropft, und das Gemisch drei Stunden bei Raumtemperatur gerührt. Man versetzt das Reaktionsgemisch danach mit 80 ml Wasser und extrahiert mit Chloroform. Die vereinigten organischen Phasen werden mit wenig Wasser gewaschen und über $Na_2SO_4$ getrocknet. Nach Abziehen des Lösungsmittels wird der kristalline Rückstand über Kieselgel mit Dichlormethan/methanol · Ammoniak (95 : 5, V/V) chromatographiert und aus Ethylacetat/Ethanol (15 : 2, V/V) kristallisiert.

Man erhält 1,4,5,6-Tetrahydro-2,6-dimethyl-4-(2-methoxyphenyl)-5-oxo-1,6-naphthyridin-3-carbonsäureethylester in Form farbloser Nadeln vom Schmp. 225 °C.

In analoger Weise werden die folgenden Verbindungen erhalten :

Die Verfahrensvariante, nach der die Verbindungen bevorzugt hergestellt werden können, sind nach dem Schmelzpunkt angegeben.

a) 1,4,5,6-Tetrahydro-6-(3-dimethylaminopropyl)-2-methyl-5-oxo-4-phenyl-1,6-naphthyridin-3-carbonsäureethylester, Schmp. 174-175 °C aus Ethylacetat ; Verf. b).

b) 1,4,5,6-Tetrahydro-4-(3-chlorphenyl)-6-(3-dimethylaminopropyl)-2-methyl-5-oxo-1,6-naphthyridin-3-carbonsäureethylester ; Schmp. 179-182 °C aus Acetonitril, Verf. b) c).

c) 1,4,5,6-Tetrahydro-4-(3-chlorphenyl)-2-methyl-5-oxo-6-(3-piperidinopropyl)-1,6-naphthyridin-3-carbonsäureethylester, Schmp. 199-201 °C aus Acetonitril ; Verf. b) c).

d) 1,4,5,6-Tetrahydro-4-(2-chlorphenyl)-6-(3-dimethylaminopropyl)-2-methyl-5-oxo-1,6-naphthyridin-3-carbonsäureethylester, Schmp. 229-230 °C aus Acetonitril ; Verf. b).

e) 1,4,5,6-Tetrahydro-4-(2-chlorphenyl)-2-methyl-5-oxo-6-(3-piperidinopropyl)-1,6-naphthyridin-3-carbonsäureethylester · 1/2 Ethylacetat, Schmp. 205-206 °C aus Ethylacetat/Ethanol ; Verf. b).

f) 1,4,5,6-Tetrahydro-2-methyl-4-(3-nitrophenyl)-5-oxo-6-(3-piperidinopropyl)-1,6-naphthyridin-3-carbonsäureethylester ; Schmp. 187-188 °C aus Acetonitril ; Verf. a) b).

g) 1,4,5,6-Tetrahydro-2-methyl-5-oxo-4-(2-pyridyl)-1,6-naphthyridin-3-carbonsäureethylester ; Schmp. 290 °C (Z.) aus Acetonitril/Ethanol ; Verf. b).

h) 1,4,5,6-Tetrahydro-6-(3-dimethylaminopropyl)-2-methyl-5-oxo-4-(2-pyridyl)-1,6-naphthyridin-3-carbonsäureethylester ; Schmp. 154-155 °C aus Ethylacetat, Verf. b).

i) 1,4,5,6-Tetrahydro-2-methyl-5-oxo-6-(3-piperidinopropyl)-4-(2-pyridyl)-1,6-naphthyridin-3-carbonsäureethylester ; Schmp. 148-150 °C, aus Ethylacetat Verf. b).

j) 1,4,5,6-Tetrahydro-2-methyl-4-(2-nitrophenyl)-5-oxo-1,6-naphthyridin-3-carbonsäureethylester ; Schmp. 215-220 °C aus Ethanol, Verf. b).

k) 1,4,5,6-Tetrahydro-6-(3-dimethylaminopropyl)-2-methyl-5-oxo-4-(2-thienyl)-1,6-naphthyridin-3-carbonsäureethylester, Schmp. 179-180 °C aus Ethylacetat, Verf. b).

l) 1,4,5,6-Tetrahydro-6-(3-dimethylaminopropyl)-2-methyl-4-(2-nitrophenyl)-5-oxo-1,6-naphthyridin-3-carbonsäureethylester, Schmp. 235-236 °C aus Acetonitril/Diisopropylether ; Verf. b).

m) 1,4,5,6-Tetrahydro-4-(2,3-dichlorphenyl)-6-(3-dimethylaminopropyl)-2-methyl-5-oxo-1,6-naphthyridin-3-carbonsäureethylester, Schmp. 268-270 °C aus Ethylacetat/Ethanol ; Verf. b).

n) 1,4,5,6-Tetrahydro-4-(2,3-dichlorphenyl)-2,6-dimethyl-5-oxo-1,6-naphthyridin-3-carbonsäureethylester ; Schmp. 294-295 °C aus Ethylacetat/Ethanol ; Verf. b).

o) 1,4,5,6-Tetrahydro-2-methyl-5-oxo-6-(3-piperidinopropyl)-4-(2-thienyl)-1,6-naphthyridin-3-carbonsäureethylester ; Schmp. 173-174 °C aus Ethylacetat ; Verf. b).

p) 1,4,5,6-Tetrahydro-6-(3-dimethylaminopropyl)-4-(2-methoxyphenyl)-2-methyl-5-oxo-1,6-naphthyridin-3-carbonsäureethylester, Schmp. 233-235 °C (Z.) aus Ethylacetat/Ethanol ; Verf. b).

q) 1,4,5,6-Tetrahydro-6-(3-dimethylaminopropyl)-2-methyl-5-oxo-4-(2-trifluormethylphenyl)-1,6-naphthyridin-3-carbonsäureethylester ; Schmp. 235-237 °C aus Ethylacetat ; Verf. b).

r) 1,4,5,6-Tetrahydro-2-methyl-5-oxo-6-(3-piperidinopropyl)-4-(2-trifluormethylphenyl)-1,6-naphthyridin-3-carbonsäureethylester ; Schmp. 204-205 °C aus Ethylacetat/Ethanol ; Verf. b).

s) 1,4,5,6-Tetrahydro-2-methyl-5-oxo-(2-trifluormethylphenyl)-1,6-naphthyridin-3-carbonsäureethylester ; Schmp. 261 °C aus Ethanol ; Verf. b).

t) 1,4,5,6-Tetrahydro-2,6-dimethyl-5-oxo-4-(2-trifluormethylphenyl)-1,6-naphthyridin-3-carbonsäureethylester ; Schmp. 258 °C aus Ethanol ; Verf. b).

u) 1,4,5,6-Tetrahydro-2,6-dimethyl-4-(3-nitrophenyl)-5-oxo-1,6-naphthyridin-3-carbonsäureethylester ; Schmp. 255-257 °C aus Ethanol ; Verf. a) b).

v) 1,4,5,6-Tetrahydro-4-(2,3-dichlorphenyl)-2-methyl-5-oxo-1,6-naphthyridin-3-carbonsäureethylester ; Schmp. 187-188 °C aus Ethanol ; Verf. b).

w) 1,4,5,6-Tetrahydro-2-methyl-5-oxo-4-(2-thienyl)-1,6-naphthyridin-3-carbonsäureethylester ; Schmp. 284-285 °C aus Ethanol ; Verf. b).

x) 1,4,5,6-Tetrahydro-2-methyl-5-oxo-4-phenyl-1,6-naphthyridin-3-carbonsäuremethylester ; Schmp. 296-300 °C (Z.) aus Methanol ; Verf. b).

y) 1,4,5,6-Tetrahydro-2-methyl-5-oxo-4-phenyl-1,6-naphthyridin-3-carbonsäure-tert.-butylester ; Schmp. 252-255 °C (Z.) aus Ethylacetat ; Verf. b).

z) 1,4,5,6-Tetrahydro-2,6-dimethyl-4-(2-nitrophenyl)-5-oxo-1,6-naphthyridin-3-carbonsäureethylester ; Schmp. 282-283 °C ; Verf. b).

a.a) 2-Ethyl-1,4,5,6-tetrahydro-5-oxo-4-phenyl-1,6-naphthyridin-3-carbonsäureethylester ; Schmp. 144-146 °C ; Verf. b).

a.b) 4-(2,3-Dichlorphenyl)-1,4,5,6-tetrahydro-2-methyl-5-oxo-6-propyl-1,6-naphthyridin-3-carbonsäureethylester ; Schmp. 264-265 °C ; Verf. b).

a.c) 1,4,5,6-Tetrahydro-2-methyl-5-oxo-4-phenyl-6-propyl-1,6-naphthyridin-3-carbonsäureethylester , Schmp. 215-217 °C ; Verf. b).

a.d) 4-(2-Chlorphenyl)-1,4,5,6-tetrahydro-2-methyl-5-oxo-1,6-naphthyridin-3-carbonsäureethylester ; Schmp. 204-205 °C (aus Ethylacetat/Ethanol) ; Verf. b).

a.e) 4-(2-Fluorphenyl)-1,4,5,6-tetrahydro-2-methyl-5-oxo-1,6-naphthyridin-3-carbonsäureethylester ; Schmp. 183-184 °C (aus Ethylacetat) ; Verf. b).

a.f) 1,4,5,6-Tetrahydro-2-methyl-4-(2-nitrophenyl)-5-oxo-1,6-naphthyridin-3-carbonsäuremethylester ; Schmp. 293-295 °C (Z) (aus Eisessig) ; Verf. b).

a.g) (±)-4-(2-Fluorphenyl)-1,4,5,6-tetrahydro-2-methyl-5-oxo-1,6-naphthyridin-3-carbonsäuremethylester ; Schmp. 315-316 °C (Z) (aus Methanol) ; Verf. b).

a.h) (±)-1,4,5,6-Tetrahydro-2-methyl-4-(2-nitrophenyl)-5-oxo-6-propyl-1,6-naphthyridin-3-carbonsäuremethylester ; Schmp. 274-276 °C (aus Methanol) ; Verf. b).

a.i) (±)-4-(2-Bromphenyl)-1,4,5,6-tetrahydro-2-methyl-5-oxo-1,6-naphthyridin-3-carbonsäuremethylester ; Schmp. 293-295 °C (Z) (aus Methanol) ; Verf. b).

a.j) (±)-4-(2-Fluorphenyl)-1,4,5,6-tetrahydro-2-methyl-5-oxo-1,6-naphthyridin-3-carbonsäure-tert.-butylester ; Schmp. 270 °C (Z) (aus Ethylacetat/Eisessig) ; Verf. b).

a.k) (±)-1,4,5,6-Tetrahydro-2-methyl-5-oxo-4-(2-trifluormethylphenyl)-1,6-naphthyridin-3-carbonsäuremethylester ; Schmp. 295 °C (aus Methanol) ; Verf. b).

a.l) (±)-4-(2,3-Dichlorphenyl)-1,4,5,6-tetrahydro-2-methyl-5-oxo-1,6-naphthyridin-3-carbonsäuremethylester ; Schmp. 318-320 °C (Z) (aus Methanol) ; Ver. b).

a.m) (±)-4-(2,3-Dichlorphenyl)-1,4,5,6-tetrahydro-2,6-dimethyl-5-oxo-1,6-naphthyridin-3-carbonsäuremethylester ; Schmp. 290-291 °C (aus Methanol) ; Verf. b).

a.n) (±)-4-(2-Fluorphenyl)-1,4,5,6-tetrahydro-6-isopropyl-2-methyl-5-oxo-1,6-naphthyridin-3-carbonsäuremethylester ; Schmp. 340-341 °C (Z) (aus Methanol) ; Verf. b).

a.o) (±)-4-(2-Bromphenyl)-1,4-dihydro-6-isopropyl-2-methyl-5-oxo-1,6-naphthyridin-3-carbonsäuremethylester ; Schmp. 330-331 °C (Z( (aus Methanol/Ethylacetat) ; Verf. b).

a.p) (±)-4-(3-Chlor-2-fluorphenyl)-1,4,5,6-tetrahydro-2-methyl-5-oxo-1,6-naphthyridin-3-carbonsäuremethylester ; Schmp. 224-226 °C (Z) (aus Methanol) ; Verf. b).

a.q) (±)-1,4,5,6-Tetrahydro-2-methyl-5-oxo-6-(3-piperidinopropyl)-4-(2-trifluormethylphenyl)-1,6-naphthyridin-3-carbonsäuremethylester ; Schmp. 168-170 °C (aus Ethylacetat) ; Verf. b).

a.r) (±)-4-(2-Diethylaminophenyl)-1,4,5,6-tetrahydro-2-methyl-5-oxo-1,6-naphthyridin-3-carbonsäuremethylester ; Schmp. 151-152 °C (Z) (aus Ethylacetat) ; Verf. b).

a.s) (±)-1,4,5,6-Tetrahydro-2-methyl-5-oxo-6-propyl-4-(2-trifluormethylphenyl)-1,6-naphthyridin-3-carbonsäuremethylester ; Schmp. 286-288 °C (Z) (aus Methanol) ; Verf. b).

a.t) (±)-4-(2-Chlor-6-fluorphenyl)-1,4,5,6-tetrahydro-2-methyl-5-oxo-1,6-naphthyridin-3-carbonsäuremethylester ; Schmp. 311-313 °C (Z) (aus Methanol/Wasser) ; Verf. b).

a.u) 1,4,5,6-Tetrahydro-2-methyl-4-(3-nitrophenyl)-5-oxo-1,6-naphthyridin-3-carbonsäure-(2-methoxyethyl) ester ; Schmp. 254-255 °C (aus Ethanol) ; Verf. b).

b.b) (±)-2-Amino-1,4,5,6-tetrahydro-5-oxo-4-phenyl-1,6-naphthyridin-3-carbonsäureethylester ; Schmp. 285 °C (Z) (aus Ethanol) ; Verf. b).

b.d) (±)-2-Amino-4-(fluorphenyl)-1,4,5,6-tetrahydro-5-oxo-1,6-naphthyridin-3-carbonsäureethylester ; Schmp. 260-262 °C (Z) (aus Ethanol) ; Verf. b).

b.e) (±)-1,4,5,6-Tetrahydro-2-methyl-4-(2-nitrophenyl)-5-oxo-1,6-naphthyridin-3-carbonsäure-(2-methoxyethyl)-ester ; Schmp. 265 °C (aus Eisessig) ; Verf. b).

b.f) (±)-6-(2-Ethoxyethyl)-4-(2-fluorphenyl)-1,4,5,6-tetrahydro-2-methyl-5-oxo-1,6-naphthyridin-3-carbonsäuremethylester ; Schmp. 215 °C (Z) (aus Ethylacetat) ; Verf. b).

b.g) (±)-4-(2-Fluorphenyl)-1,4,5,6-tetrahydro-2-methyl-5-oxo-1,6-naphthyridin-3-carbonsäure Natriumsalz ; Schmp. 227-228 °C (aus Methanol) ; Verf. b).

Die Verbindungen a.k), a.m), a.n) und b.d) liegen als Viertelhydrate (× 0,25 $H_2O$), die Verbindungen a.r) und b.g) als Hydrate (× $H_2O$) vor und die Verbindung a.e) enthält 1/2 Mol Ethylacetat (× 0,5 × $CH_3CO_2CH_2CH_3$).

Die folgenden Vergleichsversuche veranschaulichen die pharmakologische Wirksamkeit der Verbindungen gemäß allgemeiner Formel I :

I. Aggregationshemmende Wirkung

Kollagen- bzw. ADP-induzierte Thrombozytenaggregation in vitro (Ratte)

Der Test wurde nach der Methode von BORN (Nature 194, 927-929, 1962) durchgeführt. Als Blutspender dienten männliche Sprague Dawley SIV 50-Ratten mit einem Körpergewicht von 200 g. Die Blutentnahme geschah unter Äthernarkose aus dem retroorbitalen Venenplexus. 9 Teile Blut wurden mit 1 Teil 3,8 % (w/v) Tri-Na-Citrat-Lösung versetzt. Nach niedrigtouriger Zentrifugation des Gemisches bei Raumtemperatur wurde das plättchenreiche Plasma (PRP, entsprechend dem erythrozytenfreien Überstand) abgenommen und auf eine Standard-Konzentration von 400 000/ul eingestellt. Als Meßinstrument diente ein Universalaggregometer (Braun, Melsungen) verbunden mit einem Eppendorf-Photometer 1 100 M (Netherler und Hinz, Hamburg). Im Versuch wurden jeweils 700 ul PRP im Aggregometer 5 Minuten bei 37 °C äquilibriert und sodann mit 58 ul der Testsubstanzlösung bzw. einer 0,9 %igen NaCl-Lösung (entsprechend dem Leerwert) versetzt. Nach einer Inkubationszeit von 3 Minuten löste die Zugabe von 35 ul Kollagensuspension (Kollagenreagenz HORM®, Hormon Chemie, München) oder 20 ul

ADP-Lösung (Endkonz. im Test $1 \times 10^{-5}$ mol/l) die Aggregation aus. Ein Kompensationsschreiber registrierte in den folgenden 15 Minuten die Änderung der Transmission.

Die größte in diesem Zeitraum auftretende Änderung der Transmission wurde als Amplitude (Amp) der Aggregationskurve bezeichnet. Aggregationshemmende Substanzen bewirken eine Verminderung dieser Amplitude ; die inhibitorische Wirkung (E) einer Substanz errechnete sich nach folgender Formel :

$$E = (Amp_{Leerwert} - Amp_{Testsubst.})/Amp_{Leerwert} \times 100\ (\%)$$

Es wurden Dosis-Wirkungs-Kurven erstellt, anhand derer $IC_{50}$-Werte errechnet werden konnten. Der $IC_{50}$-Wert gibt die Endkonzentration einer Substanz im Testsystem an, die zu einer Erniedrigung der Amplitude um 50 % führt.

Ergebnisse

A : Kollagen-induzierte Thrombozytenaggregation
B : ADP-induzierte Thrombozytenaggregation
A/B : $IC_{50}$-Wert (mmol/l). Arith. M ± S.D.

| Beispiel Nr. | A | B |
|---|---|---|
| b.) | 0.16 ± 0.01 | 0.41 ± 0.05 |
| c.) | 0.06 ± 0.01 | 0.18 ± 0.01 |
| e.) | 0.16 ± 0.02 | 0.33 ± 0.02 |
| f.) | 0.07 ± 0.01 | 0.24 ± 0.01 |
| 2.) | 0.16 ± 0.01 | 0.51 ± 0.04 |
| Verapamil | 0.21 ± 0.02 | 0.56 ± 0.06 |

II. Cardiovaskuläre Wirkung

1. $Ca^{2+}$-Wirkung an der glatten Muskulatur

Etwa 10 mm lange, frisch isolierte Stücke der Taenia coli (Meerschweinchen) wurden an beiden Enden mit Fäden umschlungen und im Organbad (32 °C, Carbogen-durchperlt) mit einem mechanoelektrischen Transducer verbunden. Als mechanische Vorspannung wurden 5-10 mN (0,5-1 p) gewählt. Für die Äquilibrierung des Präparates wurde Krebs-Henseleit-Nährlösung verwendet (Ther 1965), die zur Depolarisation der Zellmembran durch $K^+$-reiche und $Ca^{++}$-freie Lösung (40 mmol) 1 KCl bei entsprechender Verringerung der NaCl-Konzentration ausgetauscht wurde. Unter Gleichgewichtsbedingungen wurde der Nährlösung 1 mmol/l $CaCl_2$ zugegeben, wodurch eine Kontraktion des Muskelpräparates ausgelöst wurde. Nach Erreichen des neuen Gleichgewichtes wurde die Prüfsubstanz dem Organbad zugegeben. Nach 10-minütiger Einwirkzeit wurde eine eventuelle inhibitorische Wirkung der Prüfsubstanz durch Zugabe von 12,5 mmol/l $CaCl_2$ in das Organbad zu kompensieren versucht.

Als Substanzeffekt wurde die prozentuale Hemmung der Kontraktsamplitude nach 10 min Einwirkzeit der Prüfsubstanz, bezogen auf die Amplitude vor Substanzgabe, errechnet. Alle Substanzen wurden an jeweils zwei Präparaten geprüft.

2. Isolierte Meerschweinchen-Vorhöfe

In einer thermostatisierten (35 °C) Präparierschale mit modifizierter Nährlösung nach Krebs-Henseleit wurde jeweils der linke Vorhof mittels einer Schere vorsichtig vom Ventrikelmyokard getrennt und ein Faden mit einer Nadel durch die Vorhofspitze gezogen und verknotet. Anschließend wurde die Basis des Vorhofes am Haken eines Präparatehalters befestigt und in ein thermostatisiertes Organbad mit Nährlösung (25 ml, 35 °C) überführt. Im Organbad wurde der Faden an der Vorhofspitze mit einem mechanoelektrischen Transducer verbunden. Die elektrische Reizung erfolgte supramaximal mit einer Frequenz vom 100 Imp/min (Reizbreite 2,5 msec), wobei der Haken des Präparatehalters eine Elektrode darstellt, während die zweite Elektrode als Feldelektrode im Präparatehalter parallel zum eingespannten Vorhof ausgebildet war.

Die Substanzen wurden in Abständen von 4 Minuten kumulativ dem Organbad zugegeben. Die angegebenen $IC_{50}$-Werte stellen Näherungswerte aus jeweils 4 Vorhöfen dar und geben die Substanzkonzentration an, die etwa zu einer 50 %igen Hemmung der Kontraktionsamplitude führte.

| Beispiel Nr. | $LD_{50}$ (mg/kg) (Maus) i.v. | i.g | Taenia coli % Hemmung ($3 \cdot 10^{-5}$ mol/l; 10 min) | li. Meerschw. Vorhof $IC_{50}$ (mol/l) |
|---|---|---|---|---|
| b) | 50 | 400 | 57 ± 7 | $3 \times 10^{-4}$ |
| c) | 35 | 600 | 44 ± 6 | $3 \times 10^{-4}$ |
| 1) | - | >1600 | 81 ± 5 | $> 10^{-4}$ |
| f) | 75 | 400 | 79 ± 3 | $> 10^{-4}$ |
| 2) | 75 | 600 | 88 ± 12 | $3 \times 10^{-4}$ |
| Verapamil | 7.6 | 163 | 100 ($10^{-6}$ mol/l) | $2 \times 10^{-6}$ |

**Patentansprüche** (für die Vertragsstaaten : BE, CH, DE, FR, GB, IT, LI, LU, NL, SE)

1. Naphthyridinon-Derivate der allgemeinen Formel I

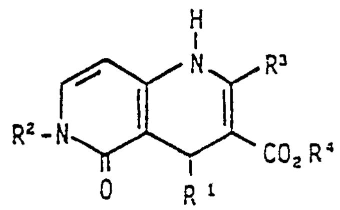

(I)

in welcher $R^1$ einen Phenylrest, der in 2- und/oder 3 Stellung durch Halogenatome, wie Fluor, Chlor oder Brom, durch Nitro-, Trifluormethyl-, Methoxy-, Difluormethoxy- oder Cyanogruppen oder Niederalkylaminogruppen, bevorzugt Dimethyl- oder Diethylaminogruppen substituiert sein kann, oder einen unsubstituierten Pyridyl- oder Thienylrest, $R^2$ Wasserstoff, eine Alkyl- oder Alkylaminoalkylgruppe der allgemeinen Formel II

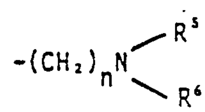

(II)

in welcher $R^5$ und $R^6$ gleich oder verschieden sein können und eine geradkettige oder verzweigte Alkylgruppe mit 1-4 C-Atomen darstellen oder gemeinsam eine Alkylengruppe mit 4-6 C-Atomen bilden, n die Zahl 2 oder 3, $R^3$ eine Aminogruppe oder eine Methyl- oder Ethylgruppe und $R^4$ Wasserstoff oder einen geradkettigen, verzweigten oder cyclischen Alkyl- oder Alkoxyalkylrest mit bis zu 6 Kohlenstoffatomen bedeutet ;
sowie gegebenenfalls deren pharmakologisch unbedenklichen Salze.

2. Verfahren zur Herstellung von 1,6-Naphthyridinon-Derivaten der allgemeinen Formel I

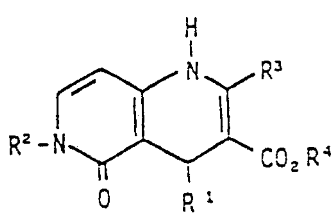

(I)

in welcher $R^1$ einen Phenylrest, der in 2- und/oder 3 Stellung durch Halogenatome, wie Fluor, Chlor oder Brom, durch Nitro-, Trifluormethyl-, Methoxy-, Difluormethoxy- oder Cyanogruppen oder Niederalkylaminogruppen, bevorzugt Dimethyl- oder Diethylaminogruppen substituiert sein kann, oder einen unsubstituierten Pyridyl- oder Thienylrest, $R^2$ Wasserstoff, eine Alkyl- oder Alkylaminoalkylgruppe der allgemeinen Formel II

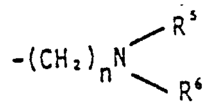

(II)

in welcher $R^5$ und $R^6$ gleich oder verschieden sein können und eine geradkettige oder verzweigte Alkylgruppe mit 1-4 C-Atomen darstellen oder gemeinsam eine Alkylengruppe mit 4-6 C-Atomen bilden, n die Zahl 2 oder 3, $R^3$ eine Aminogruppe oder eine Methyl- oder Ethylgruppe und $R^4$ Wasserstoff oder einen geradkettigen, verzweigten oder cyclischen Alkyl- oder Alkoxyalkylrest mit bis zu 6 Kohlenstoffatomen bedeutet ;
sowie von deren pharmakologisch unbedenklichen Salzen, dadurch gekennzeichnet, daß man entweder

a) 2,4-Dihydroxypyridin mit einer Verbindung der allgemeinen Formel III

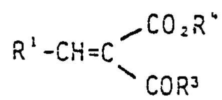

(III)

in welcher $R^1$, $R^3$ und $R^4$ die obengenannte Bedeutung haben, in Gegenwart von Ammoniak umsetzt und die so erhaltenen Verbindungen der allgemeinen Formel IV

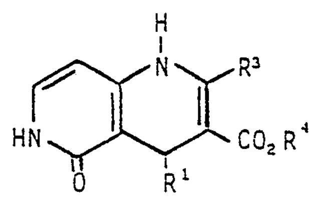

(IV)

in welcher $R^1$, $R^3$ und $R^4$ die obengenannte Bedeutung haben, gewünschtenfalls in an sich bekannter Weise mit einer Verbindung der allgemeinen Formel V

$$X—R^2, \qquad (V)$$

in welcher $R^2$ eine Alkyl- oder Alkoxyalkylgruppe oder eine Alkylaminoalkylgruppe der allgemeinen Formel II darstellt und X ein Halogenatom bedeutet, umsetzt oder

b) ein 1,4-Dihydropyridin der allgemeinen Formel

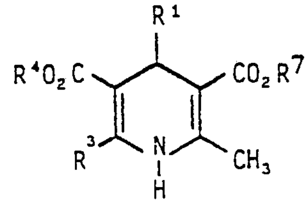

(VI)

in welcher $R^1$, $R^3$ und $R^4$ die obengenannte Bedeutung haben und $R^7$ einen Alkylrest mit 1-4 Kohlenstoffatomen bedeutet in Gegenwart einer Base mit s-Triazin umsetzt und die erhaltene Verbindung der allgemeinen Formel IV anschließend gewünschtenfalls mit einer Verbindung der allgemeinen Formel V alkyliert oder aminoalkyliert oder

c) ein 1,4-Dihydropyridin der allgemeinen Formel VI mit einem Dialkylformamid-dialkylacetal der allgemeinen Formel VII

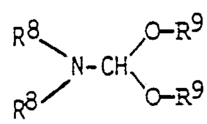

(VII)

in welcher $R^8$ gleich oder verschieden sein kann und eine Methyl- oder Ethylgruppe bedeutet und die Reste $R^9$ jeweils eine Alkylgruppe mit bis zu 4 Kohlenstoffatomen oder gemeinsam eine Alkylengruppe mit bis zu 3 Kohlenstoffatomen bedeuten
und die erhaltene Verbindung der allgemeinen Formel VIII

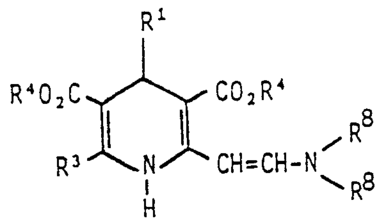

(VIII)

in welcher $R^1$, $R^3$, $R^4$ und $R^8$ die obengenannte Bedeutung haben,
mittels Ammoniak in eine Verbindung der allgemeinen Formel IV überführt und diese gewünschtenfalls mit einer Verbindung der allgemeinen Formel V alkyliert oder aminoalkyliert und die erhaltenen Verbindungen der allgemeinen Formel I ggf. durch Umsetzung mit organischen oder anorganischen Säuren in deren pharmakologisch verträglichen Salze überführt.

3. Arzneimittel zur Bekämpfung von Gefäßerkrankungen, gekennzeichnet durch einen Gehalt an mindestens einem Wirkstoff, gemäß Anspruch 1 oder 2.

**Patentanspruch** (für den Vertragsstaat AT)

Verfahren zur Herstellung von 1,6-Naphthyridinon-Derivaten der allgemeinen Formel I

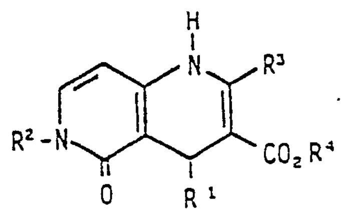

(I)

in welcher $R^1$ einen Phenylrest, der in 2- und/oder 3 Stellung durch Halogenatome, wie Fluor, Chlor oder Brom, durch Nitro-, Trifluormethyl-, Methoxy-, Difluormethoxy- oder Cyanogruppen oder Niederalkylaminogruppen, bevorzugt Dimethyl- oder Diethylaminogruppen substituiert sein kann, oder einen unsubstituierten Pyridyl- oder Thienylrest, $R^2$ Wasserstoff, eine Alkyl- oder Alkylaminoalkylgruppe der allgemeinen Formel II

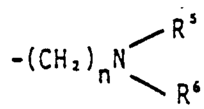

(II)

in welcher $R^5$ und $R^6$ gleich oder verschieden sein können und eine geradkettige oder verzweigte Alkylgruppe mit 1-4 C-Atomen darstellen, oder gemeinsam eine Alkylengruppe mit 4-6 C-Atomen bilden, n die Zahl 2 oder 3, $R^3$ eine Aminogruppe oder eine Methyl- oder Ethylgruppe und $R^4$ Wasserstoff oder einen geradkettigen, verzweigten oder cyclischen Alkyl- oder Alkoxyalkylrest mit bis zu 6 Kohlenstoffatomen bedeutet ;
sowie von deren pharmakologisch unbedenklichen Salzen, dadurch gekennzeichnet, daß man entweder

a) 2,4-Dihydroxypyridin mit einer Verbindung der allgemeinen Formel III

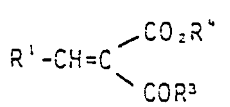

(III)

in welcher $R^1$, $R^3$ und $R^4$ die obengenannte Bedeutung haben,
in Gegenwart von Ammoniak umsetzt und die so erhaltenen Verbindungen der allgemeinen Formel IV

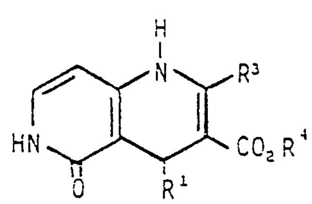

(IV)

in welcher $R^1$, $R^3$ und $R^4$ die obengenannte Bedeutung haben, gewünschtenfalls in an sich bekannter Weise mit einer Verbindung der allgemeinen Formel V

$$X—R^2, \qquad (V)$$

in welcher $R^2$, eine Alkyl- oder Alkoxyalkylgruppe oder eine Alkylaminogruppe der allgemeinen Formel II darstellt und X ein Halogenatom bedeutet, umsetzt oder

b) ein 1,4-Dihydropyridin der allgemeinen Formel

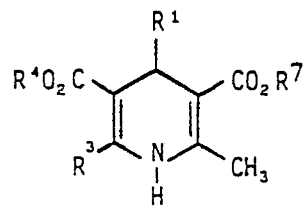

(VI)

in welcher $R^1$, $R^3$ und $R^4$ die obengenannte Bedeutung haben und $R^7$ einen Alkylrest mit 1-4 Kohlenstoffatomen bedeutet in Gegenwart einer Base mit s-Triazin umsetzt und die erhaltene Verbindung der allgemeinen Formel IV anschließend gewünschtenfalls mit einer Verbindung der allgemeinen Formel V alkyliert oder aminoalkyliert oder

c) ein 1,4-Dihydropyridin der allgemeinen Formel VI mit einem Dialkylformamid-dialkylacetal der allgemeinen Formel VII

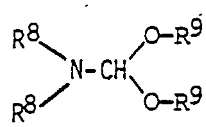

(VII)

in welcher $R^8$ gleich oder verschieden sein kann und eine Methyl- oder Ethylgruppe bedeutet und die Reste $R^9$ jeweils eine Alkylgruppe mit bis zu 4 Kohlenstoffatomen oder gemeinsam eine Alkylengruppe mit bis zu 3 Kohlenstoffatomen bedeuten
und die erhaltene Verbindung der allgemeinen Formel VIII

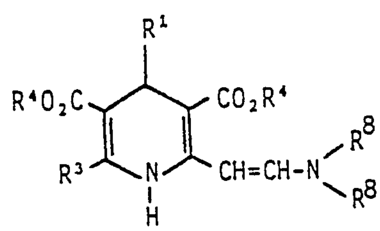

(VIII)

in welcher $R^1$, $R^3$, $R^4$ und $R^8$ die obengenannte Bedeutung haben, mittels Ammoniak in eine Verbindung der allgemeinen Formel IV überführt und diese gewünschtenfalls mit einer Verbindung der allgemeinen Formel V alkyliert oder aminoalkyliert und die erhaltenen Verbindungen der allgemeinen Formel I ggf. durch Umsetzung mit organischen oder anorganischen Säuren in deren pharmakologisch verträglichen Salze überführt.

**Claims** (for the Contracting States : BE, CH, DE, FR, GB, IT, LI, LU, NL, SE)

1. Naphthyridinone derivatives of the general formula I

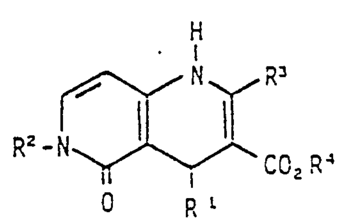

(I)

wherein $R^1$ is a phenyl radical, which in 2- and/or 3-position may be substituted by halogen atoms, such

as fluorine, chlorine or bromine, by nitro, trifluoromethyl, methoxy, difluoromethoxy, or cyano groups or lower alkylamino groups, preferably dimethylamino groups or diethylamino groups, or represents an unsubstituted pyridyl or thienyl radical, $R^2$ is hydrogen, an alkyl or alkylaminoalkyl group of the general formula II

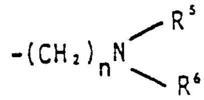

(II)

wherein $R^5$ and $R^6$ may be the same or different and represent a straight-chained or branched alkyl group with 1-4 C-atoms or represent together an alkylene group with 4-6 C-atoms, n represents the figure 2 or 3, $R^3$ is an amino group or a methyl or ethyl group and $R^4$ represents hydrogen or a straight-chained, branched or cyclic alkyl or alkoxyalkyl radical containing up to 6 carbon atoms, as well as optionally the pharmacologically safe salts thereof.

2. Process for the preparation of 1,6-naphthyridinone derivatives of the general formula I

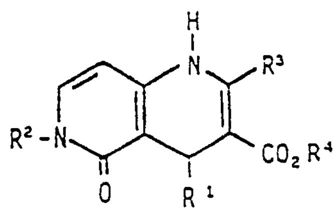

(I)

wherein $R^1$ is a phenyl radical which in 2- and/or 3-position may be substituted by halogen atoms, such as fluorine, chlorine or bromine, by nitro, trifluoromethyl, methoxy, difluoromethoxy or cyano groups or lower alkylamino groups, preferably dimethylamino groups or diethylamino groups, or represents an unsubstituted pyridyl or thienyl radical, $R^2$ is hydrogen, an alkyl or alkylamino alkyl group of the general formula II

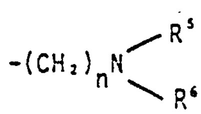

(II)

wherein $R^5$ and $R^6$ may be the same or different and represent a straight-chained or branched alkyl group with 1-4 C-atoms or represent together an alkylene group with 4-6 C-atoms, n represents the figure 2 or 3, $R^3$ is an amino group or a methyl or ethyl group and $R^4$ represents hydrogen or a straight-chained, branched or cyclic alkyl or alkoxyalkyl radical containing up to 6 carbon atoms ;
as well as the pharmacologically safe salts thereof, characterized in that either

a) 2,4-dihydroxypyridine is reacted in the presence of ammonia with a compound of the general formula III

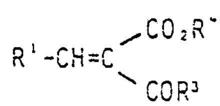

(III)

wherein $R^1$, $R^3$ and $R^4$ have the aforementioned meaning, and the compounds so obtained of the general formula IV

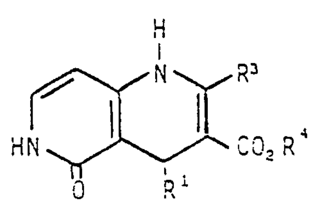

(IV)

wherein $R^1$, $R^3$ and $R^4$ have the aforementioned meaning,
are reacted, if desired, in a manner known per se with a compound of the general formula V

$$X—R^2, \quad (V)$$

wherein $R^2$ represents an alkyl or alkoxyalkyl group or an alkylamino alkyl group of the general formula II and X represents a halogen atom or

b) a 1,4-dihydropyridine of the general formula

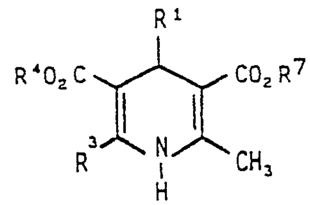

(VI)

wherein $R^1$, $R^3$ and $R^4$ have the aforementioned meaning and $R^7$ represents an alkyl radical containing 1-4 carbon atoms, is reacted with s-triazine in the presence of a base and the compound obtained of the general formula IV is, if desired, subsequently alkylated or amino-alkylated with a compound of the general formula V or

c) a 1,4-dihydropyridine of the general formula VI is reacted with a dialkylformamide-dialkyl acetal of the general formula VII

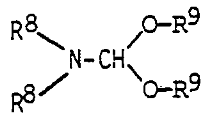

(VII)

wherein $R^8$ may be the same or different and represent a methyl or ethyl group and the radicals $R^9$ represent alkyl groups containing up to 4 carbon atoms or represent together an alkylene group containing up to 3 carbon atoms and the compound obtained of the general formula VIII

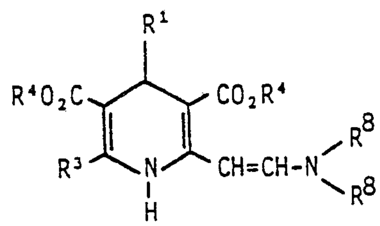

(VIII)

wherein $R^1$, $R^3$, $R^4$ and $R^8$ have the aforementioned meaning, is converted into a compound of the general formula IV by means of ammonia, alkylating or amino-alkylating it, if desired, with a compound of the general formula V and converting the compounds obtained of the general formula I into their pharmacologically acceptable salts, optionally by reaction with organic or inorganic acids.

3. A pharmaceutical preparation for fighting vascular diseases, characterised by a content of at least one active ingredient according to claim 1 or 2.

**Claim** (for the Contracting State AT)

Process for the preparation of 1,6-naphthyridinone-derivatives of the general formula I

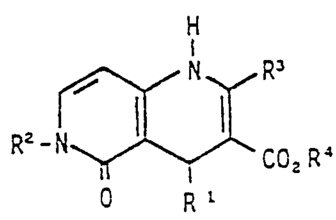

(I)

wherein $R^1$ is a phenyl radical, which in 2- and/or 3-position may be substituted by halogen atoms, such as fluorine, chlorine or bromine, by nitro, trifluoromethyl, methoxy, difluoromethoxy, or cyano groups or lower alkylamino groups, preferably dimethylamino groups or diethylamino groups, or represents an unsubstituted pyridyl or thienyl radical, $R^2$ is hydrogen, an alkyl or alkylaminoalkyl group of the general formula II

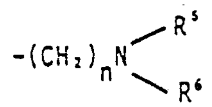 (II)

wherein $R^5$ and $R^6$ may be the same or different and represent a straight-chained or branched alkyl group with 1-4 C-atoms or represent together an alkylene group with 4-6 C-atoms, n represents the figure 2 or 3, $R^3$ is an amino group or a methyl or ethyl group and $R^4$ represents hydrogen or a straight-chained, branched or cyclic alkyl or alkoxyalkyl radical containing up to 6 carbon atoms, as well as optionally the pharmacologically safe salts thereof, characterized in that either

a) 2,4-dihydroxypyridine is reacted in the presence of ammonia with a compound of the general formula III

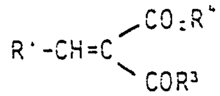 (III)

wherein $R^1$, $R^3$ and $R^4$ have the aforementioned meaning, and the compounds so obtained of the general formula IV

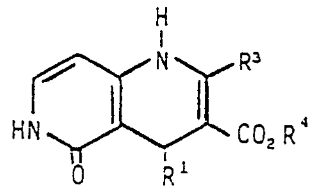 (IV)

wherein $R^1$, $R^3$ and $R^4$ have the aforementioned meaning, are reacted, if desired, in a manner known per se with a compound of the general formula V

$$X—R^2, \qquad (V)$$

wherein $R^2$ represents an alkyl or alkoxyalkyl group or an alkylamino alkyl group of the general formula II and X represents a halogen atom or

b) a 1,4-dihydropyridine of the general formula

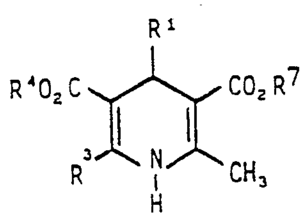 (VI)

wherein $R^1$, $R^3$ and $R^4$ have the aforementioned meaning and $R^7$ represents an alkyl radical containing 1-4 carbon atoms,
is reacted with s-triazine in the presence of a base and the compound obtained of the general formula IV is, if desired, subsequently alkylated or amino-alkylated with a compound of the general formula V or

c) a 1,4-dihydropyridine of the general formula VI is reacted with a dialkylformamide-dialkyl acetal of the general formula VII

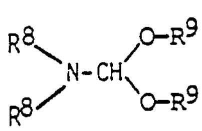 (VII)

wherein $R^8$ may be the same or different and represent a methyl or ethyl group and the radicals $R^9$ represent alkyl groups containing up to 4 carbon atoms or represent together an alkylene group containing up to 3 carbon atoms and the compound obtained of the general formula VIII

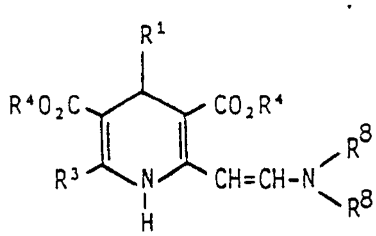 (VIII)

wherein $R^1$, $R^3$, $R^4$ and $R^8$ have to aforementioned meaning, is converted into a compound of the general formula IV by means of ammonia, alkylating or amino-alkylating it, if desired, with a compound of the general formula V and converting the compounds obtained of the general formula I into their pharmacologically acceptable salts, optionally by reaction with organic or inorganic acids.

**Revendications** (pour les Etats contractants : BE, CH, DE, FR, GB, IT, LI, LU, NL, SE)

1. Dérivés de naphtyridinone de formule générale I :

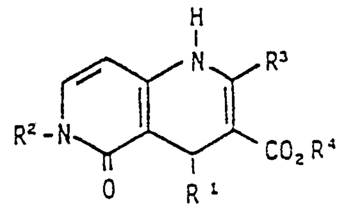

(I)

dans laquelle : $R^1$ représente un radical phényle pouvant être substitué en position 2 et/ou en position 3 par des atomes d'halogènes, tels que par exemple fluor, chlore ou brome, par des groupes nitro-, trifluorométhyl-, méthoxy-, difluorométhoxy- ou cyano-, ou des groupes alkylamino inférieurs, de préférence diméthyl- ou diéthylamino-, ou un reste pyridyl- ou thiényl- non substitué ; $R^2$ représente un atome d'hydrogène, un radical alkyle ou alkoxyalkyle renfermant jusqu'à 6 atomes de carbone ou un groupe alkylaminoalkyle de formule II :

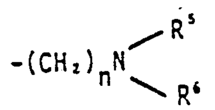

(II)

dans laquelle : $R^5$ ou $R^6$ peuvent être identiques ou différents et représentent un radical alkyle à chaîne droite ou ramifiée renfermant de 1 à 4 atomes de carbone, ou bien forment ensemble un groupe alkylène renfermant de 4 à 6 atomes de carbone ; n est égal à 2 ou 3 ; $R^3$ représente un groupe amino ou un groupe méthyle ou éthyle ; et $R^4$ représente un atome d'hydrogène ou un radical alkyle ou alkoxyalkyle à chaîne droite, ramifiée ou cyclique renfermant jusqu'à 6 atomes de carbone ;
ainsi que le cas échéant, un de leurs sels pharmaceutiquement acceptables.

2. Procédé de préparation de dérivés de 1,6-naphtyridinone de formule générale I :

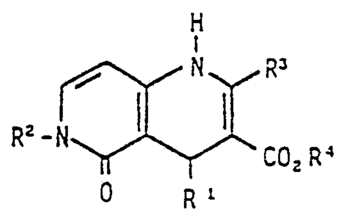

(I)

dans laquelle : $R^1$ représente un radical phényle pouvant être substitué en position 2 et/ou en position 3 par des atomes d'halogènes, tels que par exemple fluor, chlore ou brome, par des groupes nitro-, trifluorométhyl-, méthoxy-, difluorométhoxy- ou cyano-, ou des groupes alkylamino inférieurs, de préférence diméthyl- ou diéthylamino-, ou un reste pyridyl- ou thiényl- non substitué ; $R^2$ représente un atome d'hydrogène, un radical alkyle ou alkoxyalkyle renfermant jusqu'à 6 atomes de carbone ou un groupe alkylaminoalkyle de formule II :

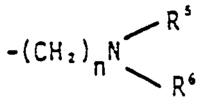

(II)

dans laquelle : $R^5$ ou $R^6$ peuvent être identiques ou différents et représentent un radical alkyle à chaîne droite ou ramifiée renfermant de 1 à 4 atomes de carbone, ou bien forment ensemble un groupe alkylène renfermant de 4 à 6 atomes de carbone ; n est égal à 2 ou 3 ; $R^3$ représente un groupe amino ou un groupe méthyle ou éthyle ; et $R^4$ représente un atome d'hydrogène ou un radical alkyle ou alkoxyalkyle à chaîne droite, ramifiée ou cyclique renfermant jusqu'à 6 atomes de carbone ;

ainsi que, le cas échéant, un de leurs sels pharmaceutiquement acceptables, caractérisé en ce que :

a) on fait réagir la 2,4-dihydroxypyridine avec un dérivé de formule générale III :

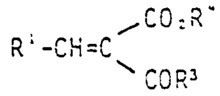

(III)

dans laquelle $R^1$, $R^3$ et $R^4$ présentent les significations ci-dessus ;
en présence d'ammoniac, et l'on fait réagir les dérivés ainsi obtenus de formule générale IV :

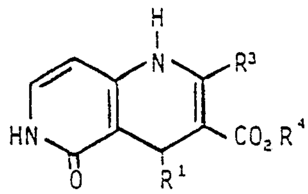

(IV)

dans laquelle $R^1$, $R^3$ et $R^4$ présentent les significations ci-dessus ;
le cas échéant, d'une façon connue en soi avec un dérivé de formule générale V :

$$X—R^2 \quad (V)$$

dans laquelle $R^2$ représente un groupe alkyle, alkoxyalkyle ou un groupe alkylamino de formule générale II ; et X signifie un atome d'halogène, ou bien

b) on fait réagir une 1,4-dihydropyridine de formule générale VI :

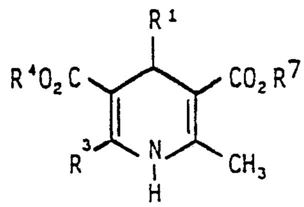

(VI)

dans laquelle : $R^1$, $R^3$ et $R^4$ présentent les significations ci-dessus ; et $R^7$ signifie un radical alkyle renfermant de 1 à 4 atomes de carbone ;
en présence d'une base avec la s-triazine et, ensuite, on procède à une alkylation ou à une aminoalkylation du dérivé obtenu de formule générale IV, le cas échéant avec un dérivé de formule générale V, ou bien

c) on fait réagir une 1,4-dihydropyridine de formule générale VI avec un dialkylformamide-dialkylacétate de formule générale VII :

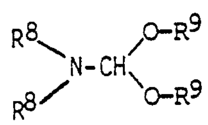

(VII)

dans laquelle : les substituants $R^8$ peuvent être identiques ou différents et signifient un radical méthyle ou éthyle ; et les radicaux $R^9$ signifient chaque fois un radical alkyle renfermant de 1 à 4 atomes de carbone, ou ils forment ensemble un groupe alkylène renfermant jusqu'à 3 atomes de carbone,
et l'on fait passer le dérivé obtenu de formule générale VIII :

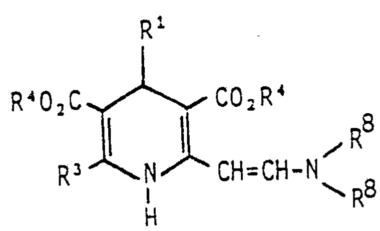

(VIII)

dans laquelle $R^1$, $R^3$, $R^4$ et $R^8$ présentent les significations indiquées ci-dessus ;
au moyen d'ammoniac, en un dérivé de formule générale IV et l'on procède à une alkylation ou à une aminoalkylation de celui-ci, le cas échéant, avec un dérivé de formule générale V, et l'on fait passer les

dérivés obtenus de formule générale I, le cas échéant par réaction avec des acides organiques ou minéraux, en leurs sels pharmaceutiquement acceptables.

3. Médicament pour lutter contre les maladies des vaisseaux, caractérisé en ce qu'il contient au moins une substance active selon la revendication 1 ou la revendication 2.

**Revendication** (pour l'Etat contractant AT)

Procédé de préparation de 1,6-naphtyridinone de formule générale I :

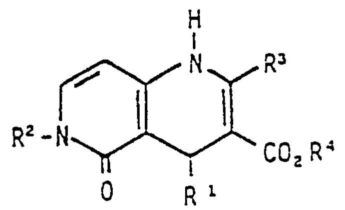

(I)

dans laquelle : $R^1$ représente un radical phényle pouvant être substitué en position 2 et/ou en position 3 par des atomes d'halogènes, tels que par exemple fluor, chlore ou brome, par des groupes nitro-, trifluorométhyl-, méthoxy-, difluorométhoxy- ou cyano-, ou des groupes alkylamino inférieurs, de préférence diméthyl- ou diéthylamino-, ou un reste pyridyl- ou thiényl- non substitué ; $R^2$ représente un atome d'hydrogène, un radical alkyle ou alkoxyalkyle renfermant jusqu'à 6 atomes de carbone ou un groupe alkylaminoalkyle de formule II :

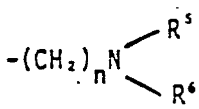

(II)

dans laquelle : $R^5$ ou $R^6$ peuvent être identiques ou différents et représentent un radical alkyle à chaîne droite ou ramifiée renfermant de 1 à 4 atomes de carbone, ou bien forment ensemble un groupe alkylène renfermant de 4 à 6 atomes de carbone ; n est égal à 2 ou 3 ; $R^3$ représente un groupe amino ou un groupe méthyle ou éthyle ; et $R^4$ représente un atome d'hydrogène ou un radical alkyle ou alkoxyalkyle à chaîne droite, ramifiée ou cyclique renfermant jusqu'à 6 atomes de carbone ;
ainsi que, le cas échéant, un de leurs sels pharmaceutiquement acceptables, caractérisé en ce que :

a) on fait réagir la 2,4-dihydroxypyridine avec un dérivé de formule générale III :

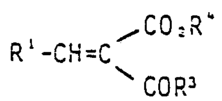

(III)

dans laquelle $R^1$, $R^3$ et $R^4$ présentent les significations ci-dessus ;
en présence d'ammoniac, et l'on fait réagir les dérivés ainsi obtenus de formule générale IV :

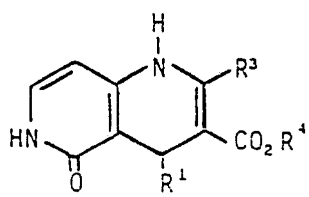

(IV)

dans laquelle $R^1$, $R^3$ et $R^4$ présentent les significations ci-dessus ;
le cas échéant, d'une façon connue en soi avec un dérivé de formule générale V :

$$X—R^2, \quad (V)$$

dans laquelle $R^2$ représente un groupe alkyle, alkoxyalkyle ou un groupe alkylamino de formule générale II ; et X signifie un atome d'halogène, ou bien

b) on fait réagir une 1,4-dihydropyridine de formule générale VI :

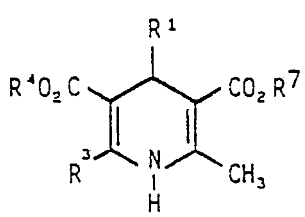

(VI)

dans laquelle : $R^1$, $R^3$ et $R^4$ présentent les significations ci-dessus ; et $R^7$ signifie un radical alkyle renfermant de 1 à 4 atomes de carbone ;
en présence d'une base avec la s-triazine et, ensuite, on procède à une alkylation ou à une aminoalkylation du dérivé obtenu de formule générale IV, le cas échéant avec un dérivé de formule générale V, ou bien

c) on fait réagir une 1,4-dihydropyridine de formule générale VI avec un dialkylformamide-dialkylacétate de formule générale VII :

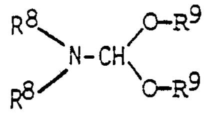

(VII)

dans laquelle : les substituants $R^8$ peuvent être identiques ou différents et signifient un radical méthyle ou éthyle ; et les radicaux $R^9$ signifient chaque fois un radical alkyle renfermant de 1 à 4 atomes de carbone, ou bien forment ensemble un groupe alkylène renfermant jusqu'à 3 atomes de carbone,
et l'on fait passer le dérivé obtenu de formule générale VIII :

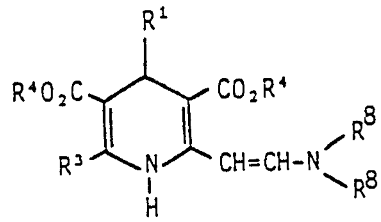

(VIII)

dans laquelle $R^1$, $R^3$, $R^4$ et $R^8$ présentent les significations indiquées ci-dessus ;
au moyen d'ammoniac, en un dérivé de formule générale IV et l'on procède à une alkylation ou à une aminoalkylation de celui-ci, le cas échéant avec un dérivé de formule générale V, et l'on fait passer les dérivés obtenus de formule générale I, le cas échéant par réaction avec des acides organiques ou minéraux, en leurs sels pharmaceutiquement acceptables.